Europäisches Patentamt

European Patent Office

Office européen des brevets

(19)

(11) Publication number: **0 251 694 B1**

(12) **EUROPEAN PATENT SPECIFICATION**

(45) Date of publication of patent specification: **04.03.92** (51) Int. Cl.⁵: **A61M 1/00**

(21) Application number: **87305650.1**

(22) Date of filing: **24.06.87**

(54) **Medical suction device.**

(30) Priority: **02.07.86 US 881374**

(43) Date of publication of application:
**07.01.88 Bulletin 88/01**

(45) Publication of the grant of the patent:
**04.03.92 Bulletin 92/10**

(84) Designated Contracting States:
**AT BE CH DE ES FR GB IT LI LU NL SE**

(56) References cited:
**EP-A- 0 158 037      WO-A-84/04684**
**FR-A- 1 476 218      US-A- 3 453 735**
**US-A- 3 645 497      US-A- 3 753 292**

(73) Proprietor: **SHERWOOD MEDICAL COMPANY**
**1915 Olive Street**
**St. Louis, MO 63103(US)**

(72) Inventor: **Kerwin, Michael John**
**826 Village Meadow Drive**
**Ballwin Missouri 63021(US)**

(74) Representative: **Porter, Graham Ronald et al**
**c/o Wyeth Laboratories Huntercombe Lane**
**South**
**Taplow Maidenhead Berkshire SL6 0PH(GB)**

## Description

This invention relates to medical suction devices and more particularly to hand-held, fine tip suckers.

Surgical suction devices, such as hand-held suckers, are used for removing blood and other body fluids from surgical sites, for example, to provide a clear view of the site during an operation. Where fine and delicate areas of the body are involved, such as when delicate brain tissue, small veins, arteries, and nerves are operated on, suckers having tips with suction orifices of small diameter are employed. These fine tips allow the accurate suctioning of very small amounts of liquid from selected areas.

With some suckers, it is possible to effect a high negative pressure at the suction orifice when the orifice is occluded by body tissue; if no liquid or air flows into the suction source the negative pressure at the suction orifice increases to that of the source. Such a high pressure differential may, of course, cause excessive tissue grab and damage to occluding body tissue.

Some suckers have suction tips of stainless steel with a suction orifice of small diameter. Such fine tips allow small quantities of liquid, such as globules of blood, to be removed from the site, However, because these tips are rigid and of small diameter, there is the danger that inadvertent movement of the manually held sucker could readily damage delicate tissue.

Some suction suckers produce relatively loud noises, such as gurgling sounds caused by the movement of air and liquid in the tip, as well as vibrations. Such vibrations are transmitted to the hand of the operator and this increases the difficulty of suctioning body fluids accurately in small surgical areas of the body.

US-A-3453735 discloses a dental aspirator tip, for use in removing saliva and ether fluids from the mouth of a patient, comprising a tubular body, an outer end on the body which is deflected laterally with reference to the remainder of the body with the outer end of the deflected portion being open, the opening therein being bounded by edges which lie in two intersecting inclined planes disposed at different angular relationships to the axis of the deflected portion of the tubular body. There is a passage through the wall of the tubular body for enabling the dentist to control the amount of suction by placing a finger over the passage opening.

It is therefore an object of the present invention to provide an improved hand-held medical suction device which reduces or substantially obviates one or more of the above mentioned problems.

According to the present invention there is provided a sucker for sucking fluid from body tissue and comprising a holder and a suction tip, said holder having a fluid passage therethrough, the proximal end of said passage being adapted for connection to suction apparatus and the distal end being connected to a tubular tip having a suction orifice at the distal end thereof, said sucker having an air bleed remote from the suction orifice and connecting the suction passage to atmosphere, characterised in that said holder has a control valve adjacent a hand holding portion and operable to close the passage, the sucker has a fluid flow restrictor in the suction passage intermediate said air bleed and control valve and the tubular tip is flexible.

The air bleed limits the maximum negative pressure at the suction orifice to values which are not damaging to body tissue should the orifice be occluded. The flexible tip ensures that inadvertent movement of the sucker is less likely to damage body tissue than hitherto.

Preferably the air bleed comprises an aperture in the wall of the tip. In a preferred embodiment the tip has a generally conical distal portion, the suction orifice being at the apex and the air bleed being adjacent the base.

The flow restrictor may be immediately adjacent the air bleed. In the preferred embodiment the air bleed has a greater cross-sectional area than the restrictor, and the restrictor has a greater cross-sectional area than the suction orifice. The flow restrictor is the most flow restrictive point of the suction passage. The suction orifice is preferably round and has a diameter less than 3.00mm and more preferably less than 1.27mm.

In the preferred embodiment the suction orifice has an approximate area of $0.85mm^2$, the air bleed has an approximate area of $6.00mm^2$ and the restrictor has an approximate area of $3.40mm^2$. The distance between the suction orifice and the air bleed is approximately 32.5mm. Such a sucker has better noise and vibration characteristics than prior art suction devices.

Other features of the invention will be apparent from the following description of a preferred embodiment shown by way of example only with reference to the accompanying drawings in which:-

Figure 1 is a side view, with portions broken away, of a hand-held suction device according to the present invention;

Figure 2 is an enlarged longitudinal cross-section through the distal end of the device of Figure 1;

Figure 3 is a view of the underside of the tip of the device of Figure 1; and

Figure 4 is a side view of a distal portion of the device of Figure 1 illustrating the flexibility of the tip.

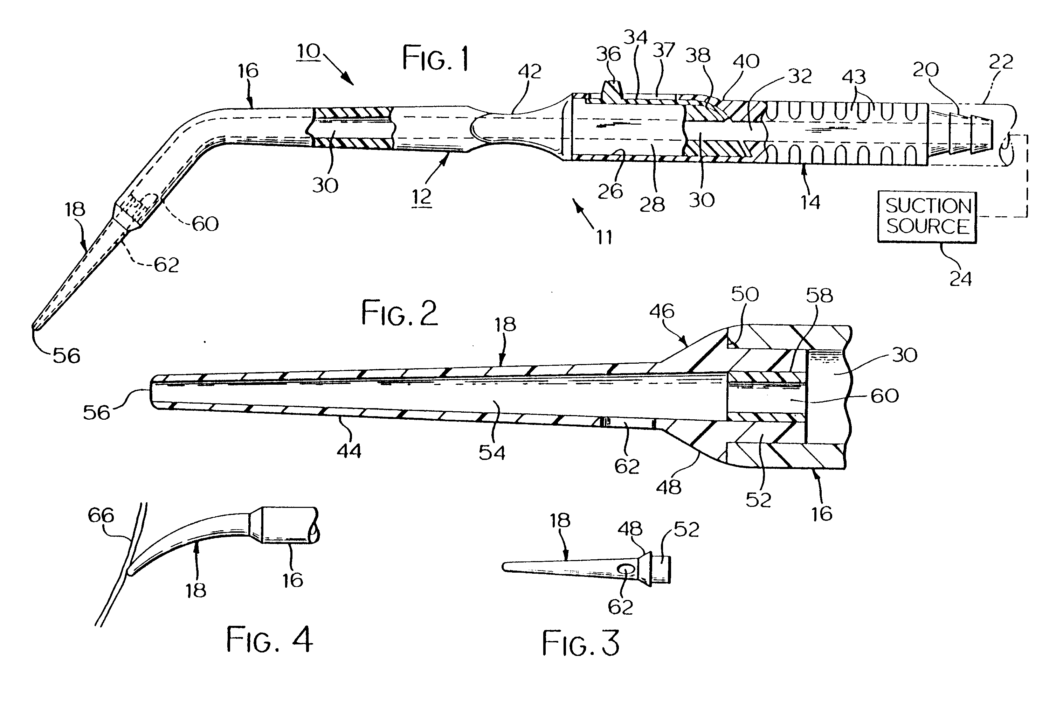

Referring now to the drawings and particularly to Figure 1, there is shown a medical suction device comprising a hand-held sucker 10 for aspirating body fluids such as blood from delicate areas of the body during surgical procedures. The device would be used in, for example, brain surgery or other operations where small and delicate body tissues, blood vessels, nerves and the like are encountered. The sucker 10 comprises a housing 11 including a hollow body member 12 having a handle 14, a probe 16, and a flexible tip 18 connected to the distal end of the probe 16. A generally conical serrated connector 20 integrally formed with handle 14 at the proximal end thereof is adapted to be connected to tubing such as indicated in phantom at 22, that connects device 10 with a suction source indicated at 24, which may include a suitable body fluid collection container.

The handle 14 has a generally rectangular socket 26 at the distal end which receives a complementary connector portion 28 of probe 16. The connector portion 28 is fixed in the socket 26, for example by a suitable adhesive. The probe 16 has an internal passageway 30 which connects with passageway 32 of the handle 14. A flexible manually operated slide valve member 34 is slidable from a position in which the distal end of probe passage 30 is open and in fluid communication with the handle passage 32, as shown in Figure 1, to a closed position in which the slide valve member shuts off the proximal end of passage 30. The valve member 34 has a finger abutment 36 which extends through an opening 37 in the upper wall of the handle 14. The proximal end of valve member 34 is slidable between complementary facing surfaces 38 and 40 of the connector portion 28 and handle 14, respectively, to close and open fluid communication between the passages 30 and 32.

Probe 16 is provided with four concave surfaces 42, one in each sidewall, so that diametrically opposed concave surfaces can be grasped between a thumb and index finger during use of the suction sucker 10; these surfaces provide a comfortable part of the device for good handling and control. The hand holding portion of the device also includes parts of the handle 14 which is shown provided with hand engaging serrations 43. The probe 16 curves downwardly so that the distal end portion extends at an obtuse angle to the longitudinal axis of the other portions of the probe for ease of handling.

The suction tip 18, as also seen in Figure 2, includes an axially extending generally conical portion 44 having an integral enlarged connector portion 46 at the proximal end. The connector portion 46 has a radially outwardly extending wall 48 ending in an annular, proximally facing flange 50 and a generally longitudinally extending tubular proximal end portion 52 that extends into the distal end of passage 30 of probe 16. The connector portion 46 of tip 18 is secured to the distal end of probe 16 by, for example, suitable adhesive. Tip 18 is provided with a suction passage 54 which is tapered or generally conical and extends radially outwardly in the proximal direction. The distal end of passage 54 comprises a suction orifice 56. The tip 18 is circular in cross-section with the diameter of the orifice 56 being smaller than that at any other point along the passage 54. The wall thickness of the tip slightly decreases from the connector portion 46 to the orifice 56. Since the main portion 44 of the tip is tapered, it provides excellent vision of small areas of the site to be aspirated.

A generally cylindrical fluid flow restrictor 58 is disposed in the proximal end portion 52 of connector 46. Restrictor 58 is secured in the tip portion 52, for example by adhesive. Restrictor 58 has an orifice 60 of smaller diameter than passage 30, but of larger diameter than suction orifice 56. The cylindrical portion 52 of tip 18 and the restrictor 58 together provide a fluid pressure differential between the suction source and passage 54 such that the suction in passage 54 will be less than the suction in passages 30 and 32 of body member 12. The restrictor 58 is the most flow restrictive point in the device including tube 22.

The sucker 10 is also provided with an air bleed or vent 62 remote from both the suction orifice 56 and the hand holding portions of the body 12. Vent 62 is formed in the sidewall of the tip 18 adjacent the proximal end thereof and also adjacent the flow restrictor 58 as shown. Vent 62 feeds ambient air into the tip passage 54 and thereby regulates suction across suction orifice 56 at a substantially constant level even when orifice 56 is completely occluded, such as by body tissue or blood. Air flow through the vent will vary as the negative pressure of the suction source 24 varies so as to maintain the suction at the suction orifice 56 substantially constant. Vent 62 is shown formed in the conical tip portion 44 at a point of relatively large diameter and is located on the bottom side of tip 18 as shown in Figures 1 to 3, that is on the inner side of the curved end portion, so that there is less chance of inadvertently occluding vent 62 by engagement with body tissue.

The flow characteristics of restrictor orifice 60, vent 62, and suction orifice 56, which are primarily determined by their respective cross-sectional areas, are so related that the suction or negative pressure at the suction tip orifice 56 is automatically maintained at a substantially constant relatively low level that cannot produce excessive tissue grab should the tip orifice 56 become occluded by body tissue. Preferably, the size of these orifices is related such as to provide a maximum grab force of, for normally expected variations in the negative pressure of the source 24, between about one-half gram and one and one-half grams, that is, such that the tip can raise a member having a weight of one-half to one and one-half grams if the member being lifted occludes the orifice 56.

A sucker made in accordance with the present invention and like that shown in the drawing had the following construction features: suction tip orifice 56 (circular): diameter 1.04mm, area 0.85mm$^2$; flow resistance orifice 60 (circular): diameter 2.08mm area 3.40mm$^2$; vent orifice 62 (elliptical): length 3.05mm width 2.54mm area 6.06mm$^2$.

In this example sucker, the largest diameter of the tip passage 54, was approximately 3.15mm and the length of the tip between flange 50 and the orifice 56 was about 37.6mm. The outer diameter of the tip at orifice 56 was about 2.41mm and the outer diameter of the tip at its junction with the enlarged connector portion 46 was about 5.33mm. The vent 62 was formed in the sidewall of the tip with the centre spaced proximally from the suction orifice 56 by approximately 32.5mm. The length of the restrictor 58 and the cylindrical proximal end portion 52 of the tip were both about 5mm and concentric with each other. The inner diameter of passage 30 of probe 16 was approximately 5.3mm as also was the tubing, such as tubing 22, connecting the device 10 to the source of suction and fluid collection device. This example device produced a suction grab force about one-half gram even though there were variations in the negative pressure of source 24.

By having the vent 62 relatively close to the suction tip orifice, only a relatively small column of liquid can be formed distally of the vent and this column can readily pass by the restriction orifice 60 and flow into the high negative pressure passage 30 and thence to the collection chamber. If the vent and flow resistance orifice were located, too far (for example, 125mm) from the suction orifice 56, it would be possible in a fine tip sucker for a substantially large column of liquid to fill that portion of the device distally of the vent and such a column might not be able to be drawn into the suction system because of the relatively low suction of that distal portion of the device. Preferably the vent is at least 12.5mm and less than 50mm from the suction orifice.

The flow resistance orifice 60 should also be as close to the vent orifice 62 and tip orifice 56 as practical so as to avoid a relatively long portion of the flow passage through the device which is at a relatively low suction. If the flow resistance orifice 60 was moved excessively proximally from the vent orifice 62 and orifice 56, there would be a considerable distance of passageway on the distal side of the resistance orifice 60 at which the negative pressure was insufficient to prevent liquid globules accumulating on the sidewall instead of quickly passing through into the higher negative pressure passageway. In such a case, the liquid and air flow in would result in gurgling sounds and vibration; such noise and vibration is reduced in the construction shown and described herein.

In the above example, the cross-sectional area of the vent 62 was approximately twice that of the restriction orifice 60 and approximately seven times that of the suction orifice 56. By making the tip and suction orifice small, the tip can be moved into small areas of the body and does not undesirably obscure the view of the operator when collecting small globules of liquid from delicate tissues. The vent 62 is spaced far enough from the orifice 56 such that it will not enter pools of blood and such that there is little chance that it will be occluded by body tissue or blood. Since the vent 62 is adjacent the enlarged connector portion 46, there is even less chance that it can be occluded during use. Also, with the vent 62 located remotely from the hand holding portions of the member 12, it cannot be inadvertently closed by a finger or hand of the operator.

The effective area of the flow restriction orifice 60 should be substantially smaller than that of the vent 62 in order to be effective in reducing suction in the tip passage 54. Because of the presence and relative size of the restriction orifice 60, the size of the vent 62 may be desirably made small enough to avoid undesirably weakening the sidewalls of the fine tip 18. Both resistance and vent orifices are of predetermined size to provide consistent operation. Also, since the vent 62 and the restriction orifice 60 are relatively close together and with the restriction orifice being only about 38mm from the suction orifice 56, only the short passageway 54 will have a relatively low negative pressure. Thus, fluids will have only a short distance to travel before reaching the passage 30 of relatively high negative pressure. In the above example, the cross-sectional area of the passage 30 was about 22.0mm$^2$ which was substantially larger than the vent and restriction orifices.

It will now be apparent that the use of the vent 62 sufficiently remote from the suction orifice 56 so that body fluid and tissue do not come in contact with the external side of vent 62, tends to regulate the suction or negative pressure differential across the suction orifice 56 upon the occurrence of an occlusion at the suction orifice 56 and thereby avoids damage to the tissue causing the occlusion.

The handle and probe may be made of a suitable, relatively rigid plastic material such as plastic resin or a copolymer and, for example, may be an acrylic copolymer. The valve slide 34 may be formed of a suitable flexible plastic such as polyethylene. The fine tip 18 is preferably formed of a flexible plastic material, such as polyvinyl chloride that is soft and flexible but which supports itself and is resilient enough to return to its original shape after being bent during use. The sidewalls of the tip 18 are normally straight, that is, without bending forces applied. Figure 4 shows that the tip 18 engaging a small body surface and being bent.

The vent 62 is shown as a single opening in the sidewall of the tip 18, it being the only opening in the tip except for those at the distal and proximal ends of the tip. The air bleed may be formed by providing one or more openings where desired.

As various changes could be made in the above construction without departing from the scope of the invention, it is intended that all matter contained in the description or shown in the accompanying drawings shall be interpreted as illustrative and not in a limiting sense.

## Claims

1. A sucker for sucking fluid from body tissue and comprising a holder (12) and a suction tip (18), said holder having a fluid passage (32) therethrough, the proximal end of said passage (32) being adapted for connection to suction apparatus (24) and the distal end being connected to a tubular tip (18) having a suction orifice (56) at the distal end thereof, said sucker having an air bleed (62) remote from the suction orifice (56) and connecting the suction passage to atmosphere, characterised in that said holder (12) has a control valve (36) adjacent a hand holding portion and operable to close the passage (32), the sucker has a fluid flow restrictor (58) in the suction passage intermediate said air bleed (62) and control valve (36) and the tubular tip (18) is flexible.

2. A sucker according to Claim 1, wherein said air bleed (62) comprises an aperture in the wall of said tip (18).

3. A sucker according to Claim 2, wherein the minimum distance between said aperture and suction orifice (56) is 12.5mm.

4. A sucker according to Claim 1 or Claim 2, wherein said tip (18) has a conical distal portion, the suction orifice (56) being at the apex of said portion and the air bleed (62) being adjacent the base.

5. A sucker according to anyone of Claims 1-4, wherein said restrictor (58) is adjacent said air bleed (62).

6. A sucker according to any one of the preceding claims, wherein said air bleed (62) has a greater cross-sectional area than said restrictor (58).

7. A sucker according to any of the preceding claims wherein said restrictor (58) has a greater cross-sectional area than said suction orifice (56).

8. A sucker according to any one of the preceding claims wherein the suction orifice (56) has an approximate area of 0.85mm$^2$, the air bleed (62) has an approximate area of 6.00mm$^2$ and the restrictor (58) has an approximate area of 3.40mm$^2$.

9. A sucker according to any of the preceding claims wherein said restrictor (58) is located in said holder.

10. A sucker according to any preceding claim, where said control valve (36) is a slide valve operable to partially close said passage to any desired extent.

11. A sucker according to any preceding claim, wherein the distal region thereof is angled away from the longitudinal axis of the hand holding portion, said air bleed (62) being located on the inner side of the angle.

12. A sucker according to any one of the preceding claims wherein the flow resistance of said restrictor (58) is greater than the flow resistance of said air bleed (62).

13. A sucker according to any one of the preceding claims, wherein said restrictor (58) is the point of maximum flow resistance upstream of said control valve (36).

## Revendications

1. Aspirateur pour aspirer du liquide de tissus corporels, comprenant un porte-embout (12) et un embout d'aspiration (18), le porte-embout présentant un passage d'écoulement (32) qui le traverse de part en part, l'extrémité proximale du passage (32) étant destinée à être raccordée à l'appareil d'aspiration (24) et son extrémité distale étant raccordée à un embout tubulaire (18) comportant un orifice d'aspiration (56) au niveau de son extrémité distale, l'aspirateur comportant un évent (62) écarté de l'orifice d'aspiration (56) et raccordant le passage d'aspiration à l'atmosphère, caractérisé en ce que le porte-embout (12) comporte une valve de commande (36) adjacente à une partie de préhension et pouvant être actionnée pour fermer le passage (32), l'aspirateur comporte un élément d'étranglement (58) du débit du liquide dans le passage d'aspiration entre l'évent (62) et la valve de commande (36) et l'embout tubulaire (18) est flexible.

2. Aspirateur suivant la revendication 1, dans lequel l'évent (62) comprend une ouverture dans la paroi de l'embout (18).

3. Aspirateur suivant la revendication 2, dans lequel la distance minimale entre l'ouverture et l'orifice d'aspiration (56) est de 12,5 mm.

4. Aspirateur suivant la revendication 1 ou 2, dans lequel l'embout (18) comporte une partie distale conique, l'orifice d'aspiration (56) étant situé au sommet de cette partie et l'évent (62) étant adjacent à sa base.

5. Aspirateur suivant l'une quelconque des revendications 1 à 4, dans lequel l'élément d'étranglement (58) est adjacent à l'évent (62).

6. Aspirateur suivant l'une quelconque des revendications précédentes, dans lequel l'évent (62) a une aire de section transversale supérieure à celle de l'élément d'étranglement (58).

7. Aspirateur suivant l'une quelconque des revendications précédentes, dans lequel l'élément d'étranglement (58) a une aire de section transversale supérieure à celle de l'orifice d'aspiration (56).

8. Aspirateur suivant l'une quelconque des revendications précédentes, dans lequel l'orifice d'aspiration (56) a une aire de section approximative de 0,85 mm², l'évent (62) a une aire de section approximative de 6,00 mm² et l'élément d'étranglement (58) a une aire de section approximative de 3,40 mm².

9. Aspirateur suivant l'une quelconque des revendications précédentes, dans lequel l'élément d'étranglement (58) est situé dans le porte-embout.

10. Aspirateur suivant l'une quelconque des revendications précédentes, dans lequel la valve de commande (36) est une valve à glissement pouvant être actionnée de manière à obturer partiellement le passage dans toute mesure souhaitée.

11. Aspirateur suivant l'une quelconque des revendications précédentes, dans lequel la région distale de celui-ci est coudée par rapport à l'axe longitudinal de la partie de préhension, l'évent (62) étant situé sur le côté intérieur de l'angle ainsi formé.

12. Aspirateur suivant l'une quelconque des revendications précédentes, dans lequel la résistance à l'écoulement de l'élément d'étranglement (58) est supérieure à la résistance à l'écoulement de l'évent (62).

13. Aspirateur suivant l'une quelconque des revendications précédentes, dans lequel l'élément d'étranglement (58) est le point de résistance maximale à l'écoulement en amont de la valve de commande (36).

## Patentansprüche

1. Saugvorrichtung zum Absaugen eines Fluids von Körpergewebe mit einem Halter (12) und einer Saugspitze (18), wobei der Halter einen Kanal (32) für das Fluid aufweist, das proximale Ende des Kanals (32) zur Verbindung mit einem Saugapparat (24) eingerichtet ist und das distale Ende mit einer röhrenförmigen Spitze (18) verbunden ist, die an ihrem distalen Ende eine Saugöffnung (56) aufweist, und wobei die Saugvorrichtung von der Saugöffnung (56) entfernt einen Nebenlufteinlaß (62) aufweist, der den Saugkanal mit der Atmosphäre verbindet,

   **dadurch gekennzeichnet,** daß

   der Halter (12) nahe einem als Handgriff ausgebildeten Abschnitt ein Stellventil (36) aufweist, das zum Verschließen des Kanals (32) betätigbar ist,

   daß die Saugvorrichtung in dem Saugkanal zwischen dem Nebenlufteinlaß (62) und dem Stellventil (36) eine Drossel (58) für den Fluidfluß aufweist, und

   daß die röhrenförmige Spitze (18) flexibel ist.

2. Saugvorrichtung nach Anspruch 1, bei der der Nebenlufteinlaß (62) ein Loch in der Wandung der Spitze (18) umfaßt.

3. Saugvorrichtung nach Anspruch 2, bei der der Abstand zwischen dem Loch und der Saugöffnung (56) mindestens 12,5 mm beträgt.

4. Saugvorrichtung nach Anspruch 1 oder 2,

bei der die Spitze (18) einen distalen konischen Abschnitt aufweist, wobei sich die Saugöffnung (56) an der Spitze dieses Abschnitts und der Nebenlufteinlaß (62) an dessen Fuß befinden.

5. Saugvorrichtung nach einem der Ansprüche 1 bis 4,
bei der die Drossel (58) dem Nebenlufteinlaß (62) benachbart ist.

6. Saugvorrichtung nach einem der vorhergehenden Ansprüche, bei der der Nebenlufteinlaß (62) eine größere Querschnittsfläche als die Drossel (58) aufweist.

7. Saugvorrichtung nach einem der vorhergehenden Ansprüche, bei der die Drossel (58) eine größere Querschnittsfläche als die Saugöffnung (56) aufweist.

8. Saugvorrichtung nach einem der vorhergehenden Ansprüche, bei der die Saugöffnung (56) eine Fläche von ungefähr 0,85 mm$^2$, der Nebenlufteinlaß (62) eine Fläche von ungefähr 6,00 mm$^2$ und die Drossel (58) eine Fläche von ungefähr 3,40 mm$^2$ aufweisen.

9. Saugvorrichtung nach einem der vorhergehenden Ansprüche, bei der sich die Drossel (58) in dem Halter befindet.

10. Saugvorrichtung nach einem der vorhergehenden Ansprüche, bei der das Stellventil (36) ein Schieberventil ist, das zum teilweisen Verschließen des Kanals, und zwar zu jedem gewünschten Grad, betätigbar ist.

11. Saugvorrichtung nach einem der vorhergehenden Ansprüche, deren distaler Teil winkelig aus der Längsachse des als Handgriff ausgebildeten Abschnitts herausgeführt ist, wobei sich der Nebenlufteinlaß (62) an der Winkelinnenseite befindet.

12. Saugvorrichtung nach einem der vorhergehenden Ansprüche, bei der der Flußwiderstand der Drossel (58) größer als der Flußwiderstand des Nebenlufteinlasses (62) ist.

13. Saugvorrichtung nach einem der vorhergehenden Ansprüche, bei der die Drossel (58) den Ort des größten Flußwiderstands in Strömungsrichtung vor dem Stellventil (36) darstellt.

FIG.1

FIG.2

FIG.4

FIG.3

EP 0 251 694 B1